# EUROPÄISCHE PATENTSCHRIFT

(11) **EP 2 118 054 B1**
(45) Veröffentlichungstag und Bekanntmachung des Hinweises auf die Patenterteilung: **06.08.2014**
(21) Anmeldenummer: 08701529.3
(22) Anmeldetag: 16.01.2008
(51) Int. Cl.: C07C 319/06, C07C 319/08, C07C 321/04

(54) **VERFAHREN ZUR HERSTELLUNG VON ALKYLMERCAPTAN AUS DIALKYLSULFIDEN UND/ODER DIALKYLPOLYSULFIDEN**
METHOD FOR PRODUCING METHYL MERCAPTAN FROM DIALKYL SULFIDES AND DIALKYL POLYSULFIDES
PROCÉDÉ DE FABRICATION DE MÉTHYLMERCAPTAN À PARTIR DE DIALKYLSULFIDES ET DE DIALKYLPOLYSULFIDES

(30) Priorität: 15.02.2007 DE 102007007458
(43) Veröffentlichungstag der Anmeldung: 18.11.2009
(73) Patentinhaber: Evonik Degussa GmbH, 45128 Essen (DE)
(72) Erfinder: BARTH, Jan-Olaf, 60598 Frankfurt (DE); REDLINGSHÖFER, Hubert, 91481 Münchsteinach (DE); WECKBECKER, Christoph, 63584 Gründau-lieblos (DE); HUTHMACHER, Klaus, 63571 Gelnhausen (DE)
(86) Internationale Anmeldenummer: PCT/EP2008/050464
(87) Internationale Veröffentlichungsnummer: WO 2008/098810

(56) Entgegenhaltungen:
- S.N. KOSHLEV, ET AL.: "Catalytic conversion of dimethylsulphide to methylmercaptan" REACTION KINETICS AND CATALYSIS LETTERS, Bd. 27, Nr. 2, September 1985 (1985-09), Seiten 387-391, XP002535032 SPRINGER SCIENCE+BUSINESS MEDIA, DORDRECHT, NL ISSN: 0133-1736

## Beschreibung

Die Erfindung betrifft ein Verfahren zur Herstellung von Alkylmercaptan durch Umsetzen einer Eduktmischung, enthaltende Dialkylsulfide und/oder -polysulfide und gegebenenfalls Dialkylether, mit Schwefelwasserstoff an heterogenen Katalysatoren.

Methylmercaptan ist ein industriell wichtiges Zwischenprodukt für die Synthese von Methionin sowie für die Herstellung von Dimethylsulfoxid und Dimethylsulfon. Methylmercaptan wird überwiegend aus Methanol und Schwefelwasserstoff durch Reaktion an einem Katalysator, bestehend aus einem Aluminiumoxidträger und Übergangsmetalloxiden und basischen Promotoren, hergestellt.

Bei der Umsetzung von Methanol mit Schwefelwasserstoff liegt bei den typischen Reaktionstemperaturen und unter Verwendung eines wirtschaftlichen Schwefelwasserstoff-Überschusses das Reaktionsgleichgewicht so, das immer Dimethylsulfid neben Methylmercaptan gebildet wird. Neben der Thioetherbildung wird auch die Reaktion zu Polysulfiden (z.B. Dimethyldisulfid) beobachtet. Diese Verbindungen werden im Zuge der Aufarbeitung des Produktgasstroms abgetrennt. Ist keine weitere wirtschaftliche Verwertung dieser Komponenten möglich, werden die Nebenprodukte üblicherweise z.B. durch Verbrennung oder Umsetzung mit Laugen entsorgt. Diese Vorgehensweise senkt die Gesamtselektivität des Herstellprozesses für Methylmercaptan und somit die Wirtschaftlichkeit des Verfahrens. Eine Alternative stellt in diesem Zusammenhang die Rückführung der Sulfide bzw. Polysulfide in den Prozess dar. Wird der Sulfidspiegel nach US 2816146 durch Recyclierung genügend hoch gehalten, so wird die Neubildung von Mercaptanen aus Alkoholen oder Ethern unterdrückt. Das Verfahren hat den gravierenden Nachteil, dass große Mengen von Sulfiden separiert, kondensiert und bei Rückführung in den Kreislauf wieder verdampft werden müssen. Dafür werden große Mengen an Wärme und Kühlenergien benötigt.

Typische Katalysatoren, die in technischen Verfahren zur Produktion von Methylmercaptan aus Methanol und Schwefelwasserstoff verwendet werden, zeigen hohe Selektivitäten für die Bildung von Methylmercaptan und führen zu einer vergleichsweise geringen Entwicklung von Dimethylsulfid und Dimethyldisulfid. Problematisch ist in diesem Zusammenhang, dass sich diese Verbindungen bei Rückführung dieser in den Prozess im Kreislauf anreichern, da durch die im Stand der Technik verwendeten Katalysatoren das Gleichgewicht zwischen Methylmercaptan und Dimethylsulfid nur schlecht eingestellt wird. Das bedeutet, dass jeweils höchstens ein Viertel des unerwünschten neu gebildeten Sulfids bei der Rückführung in den Kreislauf umgesetzt wird.

Wie die DE-C 1193038 zeigt, ist es auch möglich, das Sulfid zu separieren und in einem gesonderten Reaktionsschritt über einem anderen Katalysator zu Methylmercaptan umzusetzen. Dabei muss jedoch ein hoher Überschuss an Schwefelwasserstoff gewählt werden, um technisch relevante Umsätze an Dimethylsulfid zu erzielen. Die DE-C 1193038 beschreibt ein Verfahren, in dem man das gebildete Sulfid in einem vorgeschalteten Reaktor zusammen mit der Gesamtmenge des benötigten Schwefelwasserstoffs über einen Katalysator leitet, der das Reaktionsgleichgewicht zwischen Sulfid und Mercaptan gut einstellt (Vorkatalysator, z.B. MoO₃ / Al₂O₃) Die dabei erhaltenen Reaktionsprodukte werden anschließend, nach Zumischung von Methanol oder Dimethylether, über einen Hauptkatalysator (K₂WO₄ /Al₂O₃) geführt, an dem der Alkohol oder der Ether mit noch nicht umgesetztem Schwefelwasserstoff zu Methylmercaptan abreagiert.

Wie in der o.g. Patentanmeldung beschrieben, erweist sich die Trennung von Reaktionsprodukt und Schwefelwasserstoff bei Verwendung von großen Schwefelwasserstoffüberschüssen jedoch als schwierig.

Die JP 58159456 betrifft einen Methylmercaptan-Prozess, in dem der im Kreis geführte Schwefelwasserstoff mit frischem Schwefelwasserstoff gemischt wird und der Gesamt-H₂S-Strom anschließend zwischen einem Methylmercaptan-Reaktor und einem DMS-Spaltreaktor aufgeteilt wird. Vor dem Methylmercaptan-Reaktor wird ein H₂S-Teilstrom mit Methanol vermischt, während der zweite Teilstrom mit dem DMS in den Spaltreaktor gelangt. Die Produktströme beider Reaktoren werden anschließend gemeinsam einer Produktaufarbeitung zugeführt.

Aus der US 2831031 sind Katalysatoren auf Basis von Pyrophosphorsäure auf Titandioxid bekannt, über denen Dimethylsulfid mit einer maximalen Selektivität von 97 % bei einem Umsatz von 42 % zu Methylmercaptan umgesetzt werden. Die US 4005149 und JP 5246203 beschreiben mit Kobaltmolybdat oder Wolframsul-fid dotierte Aluminiumoxide, mit denen Dimethylsulfid-Umsätze von 41 bzw. 88% % und Selektivitäten von 92 bzw. 93 % für Methylmercaptan erzielt werden können. Als weitere Katalysatoren werden in der US 4313006 mit Natrium- oder Kalium-Ionen dotierte Zeolithe (X, Y, L) beansprucht, mit denen maximale Methylmercaptan-Selektivitäten von 65 % bei einem Dimethylsulfid-Umsatz von 70 % erzielt werden. Die JP 58159456 betrifft mit Phosphorund Wolframoxiden modifizierte Aluminiumoxide, mit denen ein maximaler DMS-Spaltumsatz von 40 % erzielt werden kann. Das H₂S/DMS-Verhältnis im Eduktgas beträgt in den o.g. Anmeldungen 2 bis 28. Vorzugsweise wird ein hohes H₂S/DMS-Verhältnis angestrebt, um ausreichend hohe DMS-Spaltumsätze zu erzielen. Die US 4005149 beschreibt ein Verfahren zur katalytischen Spaltung von organischen Sulfiden mit Schwefelwasserstoff in Anwesenheit von sülfaktiven Katalysatoren. Durch den Zusatz von Schwefelkohlenstoff zur Reaktionsmischung kann der Gesamtumsatz an Sulfiden zu Mercaptanen erhöht werden. Nachteil dieses Verfahrens ist der Einsatz von toxischem Schwefelkohlenstoff im Prozess, welcher kostenintensiv wieder von den Reaktionprodukten separiert werden muss. Generell werden bei der Spaltung von Dialkylsulfiden zu Mercaptanen mit Schwefelwasserstoff hohe Selektivitäten für Mercaptan und die weitmöglichste Unterdrückung von Nebenprodukten angestrebt. Im Gegensatz hierzu ist die Zersetzung von (Poly-) Sulfiden zu Mercaptanen, z.B. über Aluminiumoxiden, ohne den Zusatz von Schwefelwasserstoff, durch vergleichsweise niedrige Selektivitäten für Methylmercaptan und ein breites Spektrum an Nebenprodukten charakterisiert. Mashkina et al. beschreiben beispielsweise in React. Kinet. Catal. Lett., Vol. 70, No. 1, 183-189, 2000, die Zersetzung von Dimethyldisulfid zu Methylmercaptan ohne H₂S-Zusatz über sauren Katalysatoren mit maximalen Methylmercaptan-Selektivitäten von 87 %.

Nach Koshelev et al. [React. Kinet. Catal. Lett., Vol. 27, No. 2, 387-391 (1985)] wird für die Spaltung von Dimethylsulfid mit Schwefelwasserstoff über γ-Aluminiumoxid eine maximale Aktivität erzielt, wenn die Katalysatoren eine große Anzahl von aprotischen Lewis-Säurezentren und basische Zentren mit moderater Stärke aufweisen. Die von Koshelew *et al*. beschriebenen Katalysatoren auf Basis von 3,5 % Na₂O/ Al₂O₃ zeigen bei einem DMS-Umsatz von 9,5 % jedoch nur maximale Methylmercaptan-Selektivitäten von 82 %, während über reinem γ-Al₂O₃ Methylmercaptan-Selektivitäten von 97 % bei einem Umsatz von 38 % erzielt werden.

Aufgabe der Erfindung ist die Bereitstellung eines wirtschaftlichen Verfahrens, eines Apparates und von speziellen Katalysatoren zur Herstellung von Methylmercaptan aus Dialkylsulfiden und/oder -polysulfiden und Schwefelwasserstoff.

Gegenstand der Erfindung ist ein Verfahren zur kontinuierlichen Herstellung von Alkylmercaptanen durch Umsetzung eines Dialkylsulfide und/oder Dialkylpolysulfide enthaltenden Eduktgases mit einem mindestens molaren Überschuß an Schwefelwasserstoff bei erhöhter Temperatur in der Gasphase und
a) in Gegenwart eines Katalysators auf der Basis von oder bestehend aus Al₂O₃, SiO₂, TiO₂, Alumosilikaten, Zeolithen, Bentoniten oder Tonerden, die mindestens 1 Gew.-% Alkalimetalloxid enthalten,
b) in einem Reaktor, der mindestens n=2 separate Katalysatorzonen enthält, wobei man
c) den überwiegenden Teil oder die Gesamtmenge der genannten Dialkylsulfide und/oder Dialkylpolysulfide in die erste Katalysatorzone zusammen mit zumindest einem Teil der Gesamtmenge des eingesetzten Schwefelwasserstoffs in den Reaktor einleitet, und
d) die restliche Menge des Schwefelwasserstoffs und der Dialkylsulfide-und/oder Dialkylpolysulfide zwischen den Katalysatorzonen eindosiert.

Alkyl bedeutet C₁ bis C₅-Alkyl, insbesondere Methyl. Die Polysulfide besitzen im allgemeinen 2 bis 6 Schwefelatome.

Das Verfahren wird bevorzugt kontinuierlich durchgeführt.

Bevorzugte Beispiele für Dialkylsulfide und Dialkylpolysulfide, die erfindungsgemäß mit Schwefelwasserstoff zu Alkylmercaptan umgesetzt werden sind, Dimethylsulfid, Dimethyldisulfid, Dimethyltrisulfid, Dimethyltetrasulfid und Dithiapentane. Diese (Poly-) Sulfide können dem Prozess alleine oder in Mischung mit Dimethylsulfid zudosiert werden. Dem Eduktgas können auch Alkyletherverbindungen, wie z.B. Dimethylether, zudosiert werden, welche mit Schwefelwasserstoff zu Methylmercaptan umgesetzt werden.

Gleichung (1) verdeutlicht am Beispiel der Spaltung von Dimethylsulfid, dass die Reaktion ohne Bildung von Nebenprodukten durchgeführt werden kann.. Ziel der Erfindung ist, die Umwandlung zu Methylmercaptan mit einer Selektivität für das Reaktionsprodukt von größer 98 % durchzuführen. Die Bildung von weiteren Nebenprodukten, wie z.B. Schwefelkohlenstoff, soll möglichst vollständig unterdrückt werden.

CH₃-S-CH₃ + H₂S → 2 CH₃SH (1)

Bedingt durch die geringe Wärmetönung der Spaltreaktion kann das vorgeheizte Eduktgasgemisch, das Schwefelwasserstoff und Dialkyl-(Poly-) Sulfide enthält, in einem adiabaten Reaktor zu Methylmercaptan umgesetzt werden. Das Eduktgasgemisch kann auch Dialkylether oder Dialrylether enthalten, insbesonders Dimethylether.

Die Molverhältnisse von Schwefelwasserstoff und der Gesamtmenge von Dialkylsulfid und Dialkylpolysulfid belaufen sich auf 3:1 bis 25:1, bevorzugt 5:1 bis 25:1, insbesondere 10:1 bis 25:1.

Vorzugsweise wird die Reaktion in einem Reaktor durchgeführt, in dem mindestens 2, insbesondere 2 bis 25 Katalysatorzonen hintereinandergeschaltet sind. Die Katalysatorzonen können z.B als Festbetten oder Wirbelschichten oder mit Katalysator gefüllte Rohrbündel gestaltet sein. Optional können auch mehrere einzelne Apparate dieses Typs hintereinander geschaltet sein. Die gasförmige oder flüssige Dialkylsulfide und/oder -polysulfide, Schwefelwasserstoff und gegebenenfalls weitere Komponenten enthaltende Eduktmischung wird in der Weise in den Reaktor dosiert, dass bevorzugt die gesamte Menge an Dialkyl-(Poly-) Sulfiden vor der ersten Katalysatorzone mit einem Teil, der mindestens dem n-ten Teil der Gesamtmenge an Schwefelwasserstoff entspricht, zugegeben wird, während die restliche Menge Schwefelwasserstoff zwischen den Katalysatorzonen eindosiert wird. Optional kann auch die gesamte Menge Schwefelwasserstoff vor der ersten Katalysatorzone zugegeben werden.

Die Auftrennung des Produktgasgemisches kann nach verschiedenen, bekannten Verfahren erfolgen. Eine besonders vorteilhafte Auftrennung wird in der EP 0850923 B1, (US 5866721) beschrieben. Nicht umgesetztes Dialkylsulfid oder Dialkylpolysulfid wird in den Reaktor zurückgeführt.

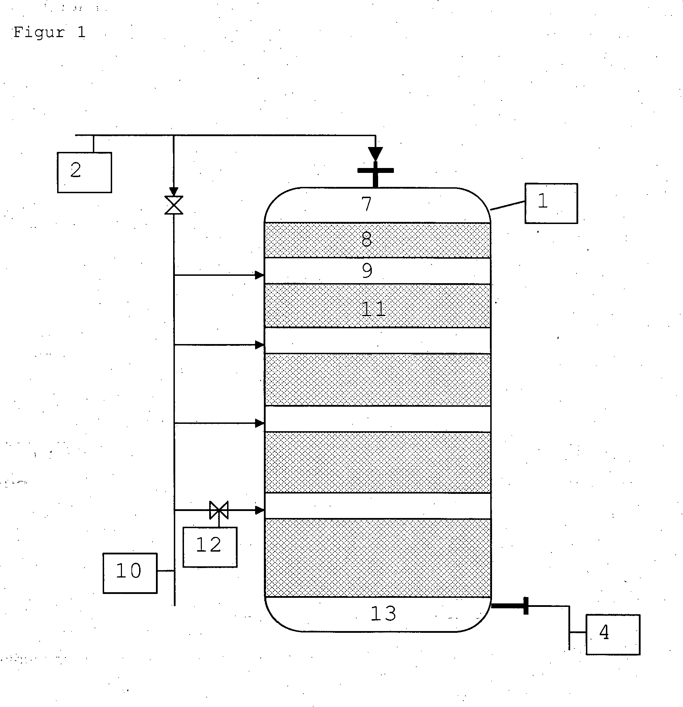

Figur 1 zeigt die bevorzugte Ausführungsform des Reaktionsapparates zur Spaltung von Dialkyl-(Poly-) Sulfiden zu Mercaptanen, gemäß Anspruch 1. Das diese Verbindungen enthaltende Gas wird als Eduktgas oder Eduktgemisch bezeichnet und stammt bevorzugt aus Verfahren zur Herstellung von Methylmercaptan aus Schwefelwasserstoff und Methanol. In dem Reaktor 1 sind n (n = 2-25) Katalysatorzonen, die aus einem Verteilerraum und einem Katalysatorbett besteht, untergebracht. Vorzugsweise werden 3-10 Katalysatorzonen verwendet. Das die genannten Alkyl-(Poly)-Sulfide enthaltende Eduktgemisch 2 tritt über den Verteilerraum 7 in das erste Katalysatorbett 8 ein. Dieses erste Katalysatorbett ist optional in Strömungsrichtung des Eduktgases zunächst mit einer Schüttung aus inerten Materialien bedeckt. Beispielsweise werden Aluminiumoxidkugeln oder Keramik-Raschigringe als Inertmaterialien verwendet. In Anschluss an die Inertschicht befindet sich die Katalysatorschüttung. Nach Verlassen der ersten Horde wird das Gasgemisch im Verteilerraum 9 mit Schwefelwasserstoff 10 oder optional dem Eduktgasgemisch 2 angereichert. Die Gasmischung strömt anschließend aus dem Verteilerraum 9 in das zweite Katalysatorbett 11, wobei Vorrichtungen im Verteilerraum 9 für eine turbulente Strömung und eine vollständige Vermischung der Reaktanden sorgen, die gleichmäßig auf die gesamte Fläche des zweiten Katalysatorbettes verteilt ist. Die Zufuhr von Schwefelwasserstoff oder optional dem Eduktgasgemisch erfolgt analog an n-1, bevorzugt (bei n>2) an n-2 Einspritzstellen zwischen den folgenden Katalysatorbetten des Apparates. Optional kann auf eine Zufuhr von Schwefelwasserstoff oder Eduktgasgemisch vor dem letzten Katalysatorbett an der Einspritzstelle 12 verzichtet werden, um einen vollständigen Umsatz in der Reaktion zu erhalten. Die letzte Katalysatorzone kann optional auch länger als die anderen Zonen gestaltet sein, um einen Vollumsatz zu ermöglichen.

Das Verfahren ist auch dadurch gekennzeichnet, dass das Eduktgas optional mindestens 0,1 Vol%, bevorzugt 0,1 bis 10 Vol%, insbesondere 1 bis 10 Vol%, Wasserstoff bezogen auf die Gesamtmenge enthält. Durch diese Maßnahmen wird die Bildung von Oligomeren und Polymeren unterdrückt. Weiterhin können im Eduktgas weitere Nebenkomponenten wie beispielsweise Stickstoff, Wasser, Kohlenmonoxid, Kohlendioxid, Carbonylsulfid oder Dialkylether enthalten sein.

Die Umsetzung der Dialkyl-(Poly-) Sulfide zu Mercaptanen erfolgt bevorzugt über Alkalimetalloxide enthaltenden Katalysatoren bei einer Temperatur von 100 bis 600°C, vorzugsweise 150 bis 450°C, insbesondere 300° bis 430°C und einem Druck von 1,5 bis 50 bar, vorzugsweise 8 bis 40 bar. Als Katalysatorträger können Silikate, Titanoxide, Zeolithe, Tonerden, Aluminiumoxide und bevorzugt γ-Aluminiumoxide eingesetzt werden. Vorzugsweise werden die Träger so mit Alkalimetalloxiden modifiziert, dass die Lewis-Acidität im Vergleich zum nicht modifizierten Katalysatorträger bei gleichzeitiger Erhöhung der Lewis-Basizität kontrolliert vermindert wird. Vorzugsweise werden 1-50Gew.-%, bevorzugt 2 bis 20 Gew.-% Alkalimetalloxid enthaltende γ-Aluminiumoxide als Katalysatoren verwendet. Bevorzugt werden Cs-Oxid oder Rb-Oxid enthaltende γ-Aluminiumoxide in dem erfindungsgemäßen Verfahren als Katalysatoren eingesetzt. Die Darstellung der Katalysatoren erfolgt z.B. über die Imprägnierung des Katalysatorträgers mit geeigneten Alkalimetallsalzen, die durch thermische Zersetzung in die entsprechende Oxide überführt werden. Vorzugsweise werden Alkalimetallnitrate, Carbonate oder die Alkalimetallsalze von Carbonsäuren verwendet. Die Katalysatoren werden anschließend getrocknet und bei Temperaturen von 50 bis 600°C optional kalziniert.

In einer besonderen Ausführungsform enthält der Katalysator oxidische Verbindungen von einem oder mehreren Übergangsmetallen der Orndungszahlen 21 bis 80, insbesondere von V, Mn, Fe, Co, Ni, Cu, Zn, Zr, Nb, Mo oder W.

Diese Metalle können auch in Form von Phosphaten oder Pyrophosphaten anwesend sein.

Vor dem erstmaligen Einsatz werden die Katalysatoren vorteilhaft mindestens 1 h bei einer Temperatur von mindestens 100°C im Schwefelwasserstoffstrom sulfidiert.

Fig. 1 zeigt eine Ausführungsform des Reaktors gemäß Anspruch 1.

In Figur 2 sind die Messergebnisse der Spaltung von Dimethylsulfid zu Methylmercaptan in Gegenwart von Schwefelwasserstoff über Katalysatoren auf Basis von Rb2O-γ-A1203, unter den Bedingungen p = 9 bar und H2S / DMS = 14 / 1 dargestellt.

In Figur 3 finden sich die Messergebnisse für die Spaltung von Dimethylsulfid zu Methylmercaptan in Gegenwart von Schwefelwasserstoff in einem Reaktor mit zwei Katalysatorzonen und H2S-Einspeisung vor den beiden Katalysatorzonen in Gegenwart verschiedener Katalysatoren. Beispiel 1 zeigt exemplarisch die Synthese der Katalysatoren auf, während in Beispiel 2 die katalytische Spaltung von Dimethylsulfid zu Methylmercaptan beschrieben wird.

### Beispiel 1:

### Präparation von M₂O-Al₂O₃ (M = Li, Na, K, Rb, Cs)

49,66 g LiNO₃ wurden in 300 ml destilliertem Wasser gelöst. Die Lösung wurde auf ca. 60°C erwärmt, so dass sich das Salz vollständig gelöst hat. Zu der Lösung wurden unter Rühren 50 g γ-Aluminiumoxid gegeben. Die Lösung wurde anschließend für ca. 60 min. gerührt. Der Katalysator wurde bei einer Temperatur von mindestens 60°C, optional bei erniedrigtem Druck, so lange gerührt bis die komplette Flüssigkeitsmenge in den Träger aufgenommen worden ist. Der Katalysator wurde über Nacht bei ca. 120°C an Luft getrocknet und anschließend bei 500°C für 3 h im Luftstrom kalziniert.

### Beispiel 2

Die DMS-Spaltung wurde in einem Temperaturbereich von 100-500°C und einem Druck von 1,5 - 25 bar untersucht. Das Verhältnis von Schwefelwasserstoff zu Dimethylsulfid (DMS) im Eduktgas wurde im Bereich von 1:1 - 25:1 variiert.

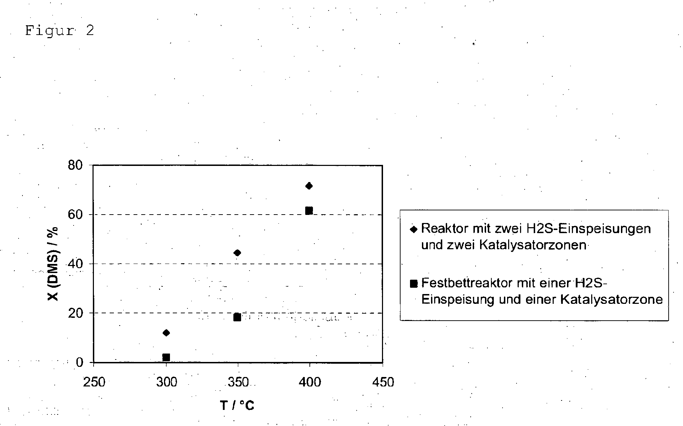

Vor Beginn der Reaktion wurden die frischen Katalysatoren zunächst im Reaktor bei 350°C für 2,5 h im Schwefelwasserstoffstrom sulfidiert. Figur 2 zeigt für Rb₂O-Al₂O₃ (Spheralith) einen Vergleich der Dimethylsulfid-Spaltumsätze zu Methylmercaptan als Funktion der Temperatur unter klassischer "Über-Kopf-Einspeisung" (Festbettreaktor mit einer Katalysatorzone)und in "Zwei-Zonen-Fahrweise", d.h. der H₂S-Strom wurde ähnlich einem Hordenreaktor mit H₂S-Zwischeneinspeisung vor den zwei Katalysatorzonen eingespeist. In beiden Fällen betrug das Gesamt-H₂S zu DMS-Verhältnis 14:1. Die Raumgeschwindigkeiten bzw. Gasbeladungen waren in beiden Fällen identisch. Figur 2 verdeutlicht, dass sich in dem erfindungsgemäßen Apparat über den in dieser Anmeldung beanspruchten, mit Alkalimetalloxiden modifizierten Katalysatoren deutlich höhere DMS-Spaltumsätze als in einer konventionellen "Einzonen-Fahrweise" z.B. in einem konventionellen Festbettreaktor, erzielen lassen.

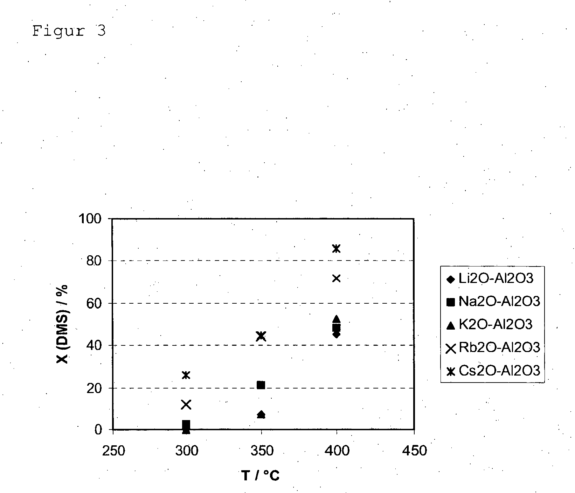

Figur 3 zeigt den positiven Einfluss der zunehmenden Lewis-Basizität der Katalysatoren in dem erfindungsgemäßen Apparat in einer "Zweizönen-Fahrweise". Mit Cs₂O-Al₂O₃-Katalysatoren wurden z.B., sowohl in "Einzonen-" und "Zweizonen-Fahrweise" deutlich höhere Spaltumsätze als mit Katalysatoren auf Basis von Li₂O-Al₂O₃ erzielt. Die Gesamtselektivität für Methylmercaptan beträgt in allen Fällen 100 %, d.h es wurden keine Nebenprodukte detektiert. Es wurden u.a. Cs₂O-Al₂O₃-Katalysatoren mit unterschiedlichen Cs₂O-Beladungen synthetisiert (5 - 10 gew.%). Wie für den Fachmann ersichtlich, können durch Modifikation hinsichtlich γ-Al₂O₃-Quelle, Durchführung der Imprägnierung, Dispersion der Alkalimetalloxide, Porosität und BET-Oberfläche des Trägers und Durchführung der Katalysatorkonditionierung bzw. Sulfidierung noch höhere DMS-Spaltumsätze erzielt werden.

Die Wirtschaftlichkeit des Gesamtprozesses hängt entscheidend von der Produktselektivität für Methylmercaptan bezogen auf die eingesetzte Kohlenstoffquelle (z.B. Methanol) ab. Aus dem Vorgenannten ist ersichtlich, dass Sulfide, wie z.B. Dimethylsulfid mit hohen Ausbeuten zu Methylmercaptan umgewandelt werden können, wodurch sich die Gesamtselektivität der Herstellung von Methylmercaptan erhöht. Ein besonderer Vorteil der Erfindung ist, dass Dialkyl-(Poly-) Sulfide, die sonst als Nebenprodukte verbrannt oder kostenintensiv entsorgt werden müssen, in einer technisch einfachen und kostengünstigen Transformation als Rohstoff für Methylmercaptan genutzt werden können. Ferner werden bei dem erfindungsgemäßen Verfahren kein toxischer Schwefelkohlenstoff oder andere Nebenprodukte gebildet.

Aus dem Produktgasgemisch wird das gebildete Methylmercaptan zusammen mit dem Methylmercaptan aus dem ersten Verfahrensschritt (z.B. Umsetzung von Methanol mit Schwefelwasserstoff), wie in DE 1768826 (GB 1268842) erläutert, in mehreren Destillations- und Wäschkolonnen bei Temperaturen zwischen 10 und 140°C abgetrennt.

## Patentansprüche

1. Verfahren zur kontinuierlichen Herstellung von Alkylmercaptanen durch Umsetzung von Dialkylsulfide und/oder Dialkylpolysulfide enthaltenden Eduktgases mit einem mindestens molaren Überschuß an Schwefelwasserstoff bei erhöhter Temperatur in der Gasphase und
a) in Gegenwart eines Katalysators auf der Basis von oder bestehend aus Al₂O₃, SiO₂, TiO₂, Alumosilikaten, Zeolithen, Bentoniten oder Tonerden, die mindestens 1 Gew.-% Alkalimetalloxid enthalten,
b) in einem Reaktor, der mindestens n=2 separate Katalysatorzonen enthält, wobei man
c) den überwiegenden Teil oder die Gesamtmenge der genannten Dialkylsulfide und/oder Dialkylpolysulfide vor der ersten Katalysatorzone zusammen mit zumindest einem Teil der Gesamtmenge des eingesetzten Schwefelwasserstoffs in den Reaktor einleitet, und
d) die restliche Menge des Schwefelwasserstoffs und der Dialkylsulfide und/oder Dialkylpolysulfide zwischen den Katalysatorzonen eindosiert,
e) wobei in der Eduktgasmischung auch Dialkylether enthalten sein können, die mit Schwefelwasserstoff zu Alkylmercaptanen reagieren und somit die Gesamtselektivität des Prozesses erhöhen.

2. Verfahren nach Anspruch 1, bei dem das Eduktgas zusätzlich Dialkylether enthält.

3. Verfahren nach Anspruch 1 oder 2, bei dem man die gesamte Menge der Dialkylsulfide und/oder
Dialkylpolysulfide in die erste Katalysatorzone zusammen mit mindestens dem n-ten Teil des eingesetzten Schwefelwasserstoffs in den Reaktor einleitet.

4. Verfahren nach Anspruch 1, 2 oder 3 bei dem man Katalysatoren, bestehend aus γ-Al₂O₃ einsetzt, das mindestens 1-Gew.-% eines Alkalimetalloxids enthält.

5. Verfahren nach den Ansprüchen 1 bis 4, bei dem man Katalysatoren einsetzt, die mindestens 1 Gew.-% eines Alkalimetalloxids, ausgewählt aus der Gruppe Cs oder Rb, enthalten.

6. Verfahren nach den Ansprüchen 1 bis 5, bei dem die Katalysatoren mit Verbindungen von Übergangsmetallen modifiziert sind.

7. Verfahren nach Anspruch 1, **dadurch gekennzeichnet, dass** die Katalysatorzonen des Reaktors als Festbetten, Rohrbündel oder Wirbelschichten gestaltet sind.

8. Verfahren nach den Ansprüchen 1 bis 7, bei dem man mehrere der Reaktoren hintereinanderschaltet.

9. Verfahren gemäß den Ansprüchen 1 bis 8, bei dem das Molverhältnis von Schwefelwasserstoff zu der Gesamtmenge von Dialkylsulfid und Dialkylpolysulfid im Bereich von 3:1 bis 25:1 1 liegt.

10. Verfahren gemäß den Ansprüchen 1 bis 9, bei dem man Dialkylsulfide und/oder Dialkylpolysulfide enthaltende Eduktgas einsetzt, das als Nebenprodukt bei der Herstellung von Alkylmercaptan anfällt.

11. Verfahren nach den Ansprüchen 1 bis 10, **dadurch**
**gekennzeichnet, dass** das Eduktgas mindestens 0,1% Wasserstoff enthält.

12. Verfahren nach den Ansprüchen 1 bis 11 **dadurch gekennzeichnet, dass** die Katalysatoren vor dem erstmaligen Einsatz für mindestens 1 h bei einer Temperatur von mindestens 100°C im Schwefelwasserstoffstrom sulfidiert werden.

13. Verfahren nach den Ansprüchen 1 bis 11, **dadurch gekennzeichnet, dass** die Umsetzung bei einer Temperatur von 100 bis 600°C, vorzugsweise 300 bis 430°C und einem Druck von 1,5 bis 50 bar, vorzugsweise 8 bis 40 bar erfolgt.

## Claims

1. Process for continuously preparing alkyl mercaptans by reacting reactant gas comprising dialkyl sulphides and/or dialkyl polysulphides with an at least molar excess of hydrogen sulphide at elevated temperature in the gas phase and
a) in the presence of a catalyst based on or consisting of Al₂O₃, SiO₂, TiO₂, aluminosilicates, zeolites, bentonites or aluminas, which contain at least 1% by weight of alkali metal oxide,
b) in a reactor which comprises at least n = 2 separate catalyst zones, wherein
c) the predominant portion or the total amount of the dialkyl sulphides and/or dialkyl polysulphides mentioned is introduced into the reactor upstream of the first catalyst zone together with at least a portion of the total amount of the hydrogen sulphide used, and
d) the remaining amount of the hydrogen sulphide and of the dialkyl sulphides and/or dialkyl polysulphides is metered in between the catalyst zones,
e) and the reactant gas mixture may also comprise dialkyl ethers which react with hydrogen sulphide to give alkyl mercaptans and hence increase the overall selectivity of the process.

2. Process according to Claim 1, in which the reactant gas additionally comprises dialkyl ethers.

3. Process according to Claim 1 or 2, in which the total amount of the dialkyl sulphides and/or dialkyl polysulphides is introduced into the reactor into the first catalyst zone together with at least the nth part of the hydrogen sulphide used.

4. Process according to Claim 1, 2 or 3, in which catalysts consisting of γ-Al₂O₃ which contains at least 1% by weight of an alkali metal oxide are used.

5. Process according to Claims 1 to 4, in which catalysts which contain at least 1% by weight of an alkali metal oxide selected from the group of Cs or Rb are used.

6. Process according to Claims 1 to 5, in which the catalysts are modified with compounds of transition metals.

7. Process according to Claim 1, **characterized in that** the catalyst zones of the reactor are configured as fixed beds, tube bundles or fluidized beds.

8. Process according to Claims 1 to 7, in which a plurality of the reactors are connected in series.

9. Process according to Claims 1 to 8, in which the molar ratio of hydrogen sulphide to the total amount of dialkyl sulphide and dialkyl polysulphide is in the range of 3:1 to 25:1.

10. Process according to Claims 1 to 9, in which reactant gas which comprises dialkyl sulphides and/or dialkyl polysulphides and is obtained as a by-product in the preparation of alkyl mercaptan is used.

11. Process according to Claims 1 to 10, **characterized in that** the reactant gas contains at least 0.1% hydrogen.

12. Process according to Claims 1 to 11, **characterized in that** the catalysts, before being used for the first time, are sulphidated at a temperature of at least 100°C in a hydrogen sulphide stream for at least 1 h.

13. Process according to Claims 1 to 11, **characterized in that** the reaction is effected at a temperature of 100 to 600°C, preferably 300 to 430°C, and a pressure of 1.5 to 50 bar, preferably 8 to 40 bar.

## Revendications

1. Procédé pour la préparation continue d'alkylmercaptans par transformation de gaz de départ contenant des sulfures de dialkyle et/ou des polysulfures de dialkyle avec au moins un excès molaire de sulfure d'hydrogène à température augmentée en phase gazeuse et
a) en présence d'un catalyseur à base de ou constitué par Al₂O₃, SiO₂, TiO₂, des aluminosilicates, des zéolithes, des bentonites ou des argiles qui contiennent au moins 1% en poids d'oxyde de métal alcalin,
b) dans un réacteur qui contient au moins n = 2 zones catalytiques séparées,
c) en introduisant la partie principale ou la quantité totale des sulfures de dialkyle et/ou des polysulfures de dialkyle mentionnés avant la première zone catalytique ensemble avec au moins une partie de la quantité totale du sulfure d'hydrogène utilisé dans le réacteur, et
d) en dosant la quantité résiduelle du sulfure d'hydrogène et des sulfures de dialkyle et/ou des polysulfures de dialkyle entre les zones catalytiques,
e) des dialkyléthers pouvant également être contenus dans le mélange de gaz de départ, qui peuvent réagir avec le sulfure d'hydrogène en alkylmercaptans et augmenter ainsi la sélectivité globale du processus.

2. Procédé selon la revendication 1, dans lequel le gaz de départ contient en outre des dialkyléthers.

3. Procédé selon la revendication 1 ou 2, dans lequel on introduit dans le réacteur la quantité totale des sulfures de dialkyle et/ou des polysulfures de dialkyle dans la première zone catalytique ensemble avec au moins la nième partie du sulfure d'hydrogène utilisé.

4. Procédé selon la revendication 1, 2 ou 3, dans lequel on utilise des catalyseurs constitués de γ-Al₂O₃, qui contient au moins 1% en poids d'un oxyde de métal alcalin.

5. Procédé selon les revendications 1 à 4, dans lequel on utilise des catalyseurs qui contiennent au moins 1% en poids d'un oxyde de métal alcalin, choisi dans le groupe formé par Cs ou Rb.

6. Procédé selon les revendications 1 à 5, dans lequel les catalyseurs sont modifiés par des composés de métaux de transition.

7. Procédé selon la revendication 1, **caractérisé en ce que** les zones catalytiques du réacteur sont conçues comme lits fixes, faisceaux tubulaires ou couches tourbillonnantes.

8. Procédé selon les revendications 1 à 7, dans lequel on commute plusieurs réacteurs les uns derrière les autres.

9. Procédé selon les revendications 1 à 8, dans lequel le rapport molaire de sulfure d'hydrogène à la quantité totale de sulfure de dialkyle et de polysulfure de dialkyle se situe dans la plage de 3:1 à 25:1.

10. Procédé selon les revendications 1 à 9, dans lequel on utilise un gaz de départ contenant des sulfures de dialkyle et/ou des polysulfures de dialkyle qui est obtenu comme produit secondaire lors de la préparation d'alkylmercaptan.

11. Procédé selon les revendications 1 à 10, **caractérisé en ce que** le gaz de départ contient au moins 0,1% d'hydrogène.

12. Procédé selon les revendications 1 à 11, **caractérisé en ce que** les catalyseurs sont sulfurés avant la première utilisation, pendant au moins 1 h à une température d'au moins 100°C, dans un flux de sulfure d'hydrogène.

13. Procédé selon les revendications 1 à 11, **caractérisé en ce que** la transformation est réalisée à une température de 100 à 600°C, de préférence de 300 à 430°C, et à une pression de 1,5 à 50 bars, de préférence de 8 à 40 bars.
